# FASCICULE DE BREVET EUROPEEN

(11) **EP 4 622 601 B1**
(45) Date de publication et mention de la délivrance du brevet: **14.10.2026**
(21) Numéro de dépôt: 23814234.3
(22) Date de dépôt: 06.11.2023
(51) Int. Cl.: A61F 2/44, A61F 2/46

(54) **CAGE APTE À ÊTRE IMPLANTÉE ENTRE DEUX PORTIONS OSSEUSES**
KÄFIG ZUR IMPLANTATION ZWISCHEN ZWEI KNOCHENTEILEN
CAGE SUITABLE FOR BEING IMPLANTED BETWEEN TWO BONE PORTIONS

(30) Priorité: 22.11.2022 FR 2212132
(43) Date de publication de la demande: 01.10.2025
(73) Titulaire: Razian, Hassan, 94240 L'Hay-les-Roses (FR)
(72) Inventeur: RAZIAN, Sam, 94240 L'HAY les ROSES (FR); RAZIAN, Salar, 94240 L'HAY les ROSES (FR); RAZIAN, Hassan, 94240 L'HAY les ROSES (FR)
(74) Mandataire: Boüan du Chef du Bos, Louis-Paterne
(86) Numéro de dépôt international: PCT/FR2023/000171
(87) Numéro de publication internationale: WO 2024/110702

(56) Documents cités:
- FR-A1- 2 955 483
- US-A1- 2009 265 007
- US-A1- 2014 163 682
- US-A1- 2015 305 880
- US-B1- 8 545 562

## Description

La présente invention concerne les cages aptes à être implantées entre deux portions osseuses, qui trouvent une application particulièrement avantageuse comme cages intersomatiques prévues pour être implantées entre deux vertèbres consécutives.

On connaît déjà des cages aptes à être interposées au contact entre deux portions osseuses.

Ces cages comprennent un corps solide délimité par deux faces d'appui opposées assimilables à deux surfaces planes sensiblement parallèles entre elles, ces deux faces d'appui étant agencées pour être aptes à venir au contact respectivement des deux portions osseuses, ainsi que, généralement située dans une cavité réalisée dans ce corps solide, au moins une lame couplée à des moyens pour la faire pivoter de façon que, dans une position de repos, elle soit entièrement contenue dans la cavité et que, à la suite d'une rotation par rapport au corps solide, elle émerge au moins partiellement de cette cavité pour venir s'ancrer dans la ou les portions osseuses et assurer la fixation potentiellement définitive de la cage par rapport à ces portions osseuses.

Une telle cage est par exemple connue du US 2009/265007. Cette cage donne satisfaction dans la grande majorité des cas, mais peut présenter l'inconvénient constitué par le fait que, lorsque les os, comme les vertèbres entre lesquelles elle a été implantée, bougent, il peut se produire des micromouvements verticaux qui risquent d'induire des effets néfastes pour la personne sur laquelle a été implantée une telle cage. D'autres exemples de cage de fusion équipée de lames d'ancrages rotatives sont décrits dans les brevets US 2014/163682 A1, US 2015/305880 A1 et FR 2 955 483 A1.

Aussi, la présente invention a-t-elle pour but de réaliser une cage qui, tout en présentant une structure relativement simple et de réalisation peu onéreuse, tente de répondre aux éventuels souhaits des Praticiens quant à sa stabilité tout en facilitant le travail de ces Praticiens pour son implantation.

Plus précisément, la présente invention a pour objet une cage intersomatique apte à être implantée entre deux portions osseuses, telle que définie dans au moins l'une des revendications annexées.

D'autres caractéristiques et avantages de la présente invention apparaîtront au cours de la description suivante donnée en regard des dessins annexés à titre illustratif, mais nullement limitatif, dans lesquels :
La [Fig 1] est une vue très schématique et en perspective cavalière, d'un mode de réalisation de la cage selon l'invention, et
La [Fig 2] est une vue schématique de dessus, mais plus détaillée, du mode de réalisation de la cage selon la figure 1.

Il est précisé que, dans la présente description, si l'adverbe "sensiblement" est associé à un qualificatif d'un moyen donné, ce qualificatif doit être compris au sens strict ou approché.

La cage intersomatique selon l'invention est apte à être implantées entre deux portions osseuses, par exemple de façon très avantageuse entre deux corps vertébraux consécutifs.

La cage comprend un corps solide 10 qui est délimité par deux faces d'appui opposées 13, 14 reliées par une paroi latérale 15, ces faces d'appui étant assimilables, pour la facilité de la description, à des surfaces planes sensiblement parallèles entre elles, même si elles forment entre elles une légère angulation. Elles sont en outre agencées pour être aptes à venir au contact respectivement des deux portions osseuses, comme pour les cages de l'art antérieur.

Le corps solide 10 comporte une cavité traversante 11 débouchant respectivement à ses deux extrémités sur les deux faces d'appui 13, 14 de façon que l'axe sensiblement central 12 de la cavité 11 soit de préférence perpendiculaire à ces deux faces d'appui (compte tenu de l'assimilation définie ci-dessus).

La cage comprend aussi des moyens pour solidariser le corps solide avec au moins l'une des deux portions osseuses, de préférence avec les deux portions osseuses dans la majorité des cas d'application.

Selon la présente invention, les moyens pour solidariser le corps solide 10 avec au moins l'une des deux portions osseuses comprennent au moins une plaque support 20 ; des moyens pour monter la plaque support dans la cavité 11 en coopération avec le corps solide ; et au moins une lame d'ancrage 30.

Sont aussi prévus des moyens pour monter la lame d'ancrage 30 en coopération de déplacement sur la plaque support de façon que, dans une première position, la lame d'ancrage 30 soit entièrement contenue dans la cavité 11 et que, dans une seconde position après son déplacement par rapport à la plaque support 20, au moins une partie 31, 32 de la lame d'ancrage 30 émerge de la cavité 11.

Selon une caractéristique avantageuse de l'invention, les moyens pour monter la plaque support 20 dans la cavité 11 en coopération avec le corps solide 10 sont constitués par des moyens pour la fixer sur le corps solide, cette plaque support 20 étant en outre agencée de façon à être apte à prendre différentes orientations angulaires dans la cavité par rapport au corps solide 10, de façon avantageuse selon au moins l'une des deux rotations suivantes : une rotation autour de l'axe central 12, une rotation autour d'une droite sensiblement perpendiculaire à cet axe central et perpendiculaire à l'axe de rotation de la lame d'ancrage par rapport à la plaque support.

Les moyens cités ci-dessus peuvent être de tout type, par exemple des vis ou analogues. Ils sont du domaine de l'homme de l'art qui sera en outre guidé, pour leur mise en oeuvre selon son choix, par les enseignements des deux figures annexées. Selon une autre caractéristique de l'invention, les moyens pour monter la lame d'ancrage 30 en coopération de déplacement sur la plaque support 20 sont constitués par des moyens de déplacement en rotation 40 de cette lame d'ancrage par rapport à la plaque support.

Selon une caractéristique avantageuse et préférentielle de l'invention, les moyens de déplacement en rotation 40 de la lame d'ancrage 30 par rapport à la plaque support 20 comportent un arbre 41 couplé avec la plaque support et des moyens pour monter la lame d'ancrage en coopération de rotation avec cet arbre 41.

De façon avantageuse, cet arbre 41 est monté en rotation dans la plaque support 20 et les moyens pour monter la lame d'ancrage 30 en coopération avec l'arbre 41 sont agencés de façon que la lame soit solidarisée avec l'arbre 41. De ce fait, l'arbre et la lame pivotent ensemble, ce qui confère à l'ensemble « plaque 20-arbre 41-lame 30 » une bonne rigidité. Il est même avantageux, pour obtenir le résultat défini ci-dessus, que la lame d'ancrage 30 soit montée en rotation sur la plaque support 20 de façon serrée.

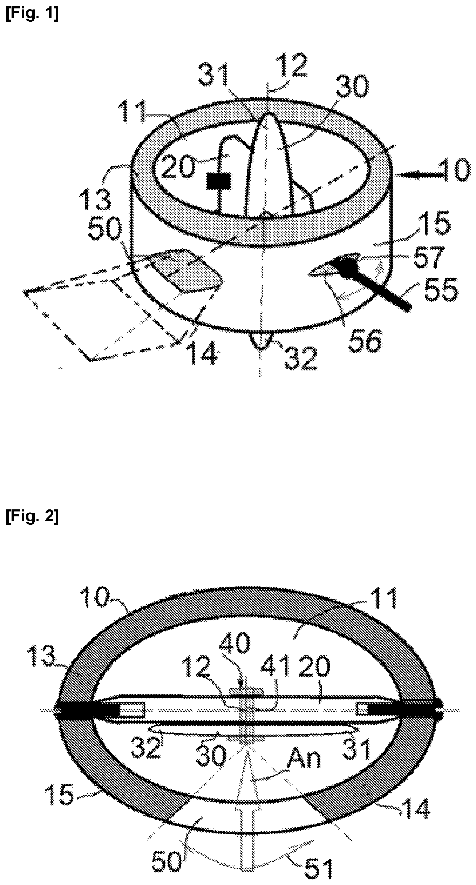

En outre, préférentiellement, la lame d'ancrage 30 est configurée pour que, dans sa seconde position, elle comporte deux parties 31, 32, à savoir ses deux extrémités, qui émergent respectivement des deux extrémités la cavité 11, comme évoqué figure 1.

Selon une autre caractéristique de l'invention, la paroi du corps solide comporte un orifice 50 débouchant dans la cavité 11, cet orifice ayant une section apte à laisser passer un outil ancillaire An pour au moins commander le passage de la lame d'ancrage de l'une de ses deux positions à l'autre. Mais cette section peut aussi avoir une valeur déterminée de façon à permettre à l'ancillaire de prendre différentes positions angulaires pour donner à la plaque et donc à la lame, la position angulaire voulue par rapport aux deux portions osseuses, avant de faire tourner la lame pour l'implanter dans la ou les portions osseuses, comme il sera explicité ci-après dans un exemple d'utilisation possible de la cage selon l'invention.

La section de cet orifice 50 sera définie par l'homme de l'art de façon que l'ancillaire puisse prendre différentes orientations 51 par rapport à l'axe central 12 de la cavité 11, par exemple dans un cône comme évoqué sur la figure 1, pour obtenir les bonnes implantations.

Sont en outre prévus des moyens pour fixer la plaque support quand elle a pris une orientation déterminée. Ces moyens peuvent être de tous types connus des hommes du métier, par exemple constitués d'une crémaillère anti-retour ou analogue évoquée schématiquement en 55 sur la figure 1, avec des moyens pour la rendre solidaire du corps 10, par exemple par coincement dans l'orifice oblong 56. La tige de commande de la crémaillère montée en coopération avec la plaque 20 peut être désolidarisée de la crémaillère, après implantation de la cage comme décrit ci-après, au moyen du découpleur 57, figure 1.

Sur la cage illustrée sur les deux figures, il n'existe qu'un seul couple plaque/lame. Mais il est aussi possible que la cage comporte au moins deux couples « plaque support/lame d'ancrage 20/30 » situés dans la cavité 11, dans deux positions non confondues et avec au moins une lame sur chaque plaque.

A titre d'exemple de réalisation préférentiel, mais non limitatif : le corps 10 peut être réalisé en PEEK, à savoir en Polyétherétherketone ; la lame d'ancrage 30 et la plaque support 20 peuvent être réalisées en un alliage de titane du type TA6V-ELI à base d'aluminium-vanadium.

La cage selon l'invention fonctionne et s'utilise de la façon suivante décrite ici, à titre d'exemple, dans une réduction d'un spondylolisthésis.

Lorsque le Praticien a fait son diagnostic et qu'il a déterminé la cage, à savoir le couple « plaque/lame », qu'il estime nécessaire d'utiliser, il configure la cage de façon que la lame 30 soit située, par rapport à la plaque 20, dans sa première position définie ci-avant et représentée sur la figure 2.

Il introduit la cage ainsi préparée, à l'aide de l'ancillaire An, entre les deux vertèbres qui sont à l'origine du spondylolisthésis. Puis, toujours à l'aide de l'ancillaire, il oriente la plaque (qui porte la lame toujours dans sa première position) en la faisant pivoter selon les possibilités comme définies ci-avant, la lame demeurant toujours, lors de ces manipulations, dans sa première position.

Quand il estime que le couple « plaque/lame » est dans une position qui peut régler le problème siège du mal, il fixe définitivement la position de la plaque, par exemple au moyen de la crémaillère anti-retour, puis fait pivoter la lame 30 pour l'amener dans sa seconde position de façon que ses deux extrémités 31, 32 s'implantent respectivement dans les parties osseuses des deux vertèbres (figure 1), comme avec les cages de l'art antérieur.

L'avantage principal de la structure de cette cage selon l'invention est de permettre de choisir une position angulaire de la lame par rapport au corps 10, via l'orientation de la plaque, après que la cage ait été introduite entre les vertèbres (avant, bien entendu, de faire pivoter la lame par rapport à la plaque), ce qui était impossible d'obtenir avec les cages de l'art antérieur.

Il est à noter que, dans l'exemple décrit ci-dessus, la cage ne comporte qu'un seul couple « plaque20/lame30 ». Il est cependant précisé que la cage peut comporter, par exemple, deux couples pour obtenir deux lames d'ancrage ayant des orientations angulaires différentes, de façon à réaliser un effet de coin bien connu en lui-même pour obtenir un parfait maintien des deux vertèbres.

Les caractéristiques structurelles de la cage selon l'invention soulignées ci-dessus permettent d'assurer un ancrage de la cage dans les deux portions d'os, de façon encore plus parfaite que celle des cages de l'art antérieur, ce qui est primordial pour un patient, tout en facilitant le travail d'implantation d'une telle cage.

## Revendications

1. Cage intersomatique apte à être implantée entre deux portions osseuses, ladite cage comprenant un corps solide (10) délimité par deux faces d'appui opposées (13, 14) reliées par une paroi latérale de surface extérieure (15), ces faces d'appui étant assimilables à des surfaces planes sensiblement parallèles entre elles et étant agencées pour être aptes à venir au contact respectivement des deux portions osseuses, ledit corps solide (10) comportant en outre une cavité traversante (11) débouchant respectivement à ses deux extrémités sur les deux faces d'appui (13, 14) de façon que l'axe central (12) de la cavité (11 ) soit sensiblement perpendiculaire à ces deux faces d'appui ; et des moyens pour solidariser ledit corps solide avec au moins l'une des deux portions osseuses, ces moyens pour solidariser ledit corps solide (10) avec au moins l'une des deux portions osseuses comprenant : au moins une plaque support (20) ; des moyens pour monter ladite plaque support dans ladite cavité (11 ) en coopération avec ledit corps solide ; au moins une lame d'ancrage (30) ; et des moyens pour monter ladite lame d'ancrage en coopération de déplacement sur ladite plaque support constitués par des moyens de déplacement en rotation (40) de ladite lame d'ancrage par rapport à ladite plaque support, de façon que, dans une première position, ladite lame d'ancrage (30) soit entièrement contenue dans ladite cavité (11 ) et que, dans une seconde position après son déplacement par rapport à ladite plaque support (20), au moins une partie (31 , 32) de ladite lame d'ancrage (30) émerge de ladite cavité (11 ), les moyens pour monter ladite plaque support (20) dans ladite cavité (11 ) en coopération avec ledit corps solide (10) étant constitués par des moyens pour fixer ladite plaque support (20) sur ledit corps solide, ladite plaque support (20) étant en outre agencée de façon à être apte à prendre différentes orientations angulaires dans ladite cavité par rapport au corps solide (10), selon au moins l'une des deux rotations suivantes : une rotation autour de l'axe central (12), une rotation autour d'une droite sensiblement perpendiculaire à cet axe central et perpendiculaire à l'axe de rotation de la lame d'ancrage par rapport à la plaque support.

2. Cage selon la revendication 1, **caractérisée par le fait que** les moyens de déplacement en rotation (40) de la lame d'ancrage (30) par rapport à la plaque support (20) comportent un arbre (41) couplé avec ladite plaque support et des moyens pour monter ladite lame d'ancrage en coopération de rotation avec ledit arbre.

3. Cage selon la revendication 2, **caractérisée par le fait que** ledit arbre (41) est monté en rotation dans la plaque support (20) et que les moyens pour monter ladite lame d'ancrage (30) en coopération avec ledit arbre (41) sont agencés de façon que ladite lame soit solidarisée avec ledit arbre (41).

4. Cage selon la revendication 3, **caractérisée par le fait que** ladite lame d'ancrage (30) est montée en rotation sur ladite plaque support (20) de façon serrée.

5. Cage selon l'une des revendications précédentes, **caractérisée par le fait que** ladite lame d'ancrage (30) est configurée pour que, dans sa seconde position, au moins deux parties (31, 32) de ladite lame émergent respectivement des deux extrémités ladite cavité (11).

6. Cage selon l'une des revendications précédentes, **caractérisée par le fait que** la paroi du dit corps solide comporte un orifice (50) débouchant dans ladite cavité (11 ), ledit orifice ayant une section apte à laisser passer un outil ancillaire (An) pour commander le passage de ladite lame d'ancrage de l'une de ses deux positions à l'autre, la section dudit orifice (50) ayant une valeur déterminée de façon que l'ancillaire puisse prendre différentes orientations (51 ) par rapport audit axe central (12) de ladite cavité (11).

7. Cage selon la revendication 6, **caractérisée par le fait qu'**elle comporte des moyens pour fixer ladite plaque support quand elle a pris une orientation déterminée.

8. Cage selon la revendication 7, **caractérisée par le fait que** les moyens pour fixer ladite plaque support (20) quand elle a pris une orientation déterminée sont constitués par une crémaillère anti-retour.

9. Cage selon l'une des revendications précédentes, **caractérisée par le fait qu'**elle comporte au moins deux couples « plaque support/lame d'ancrage (20/30) » situés dans ladite cavité (11) dans deux positions non confondues.

## Patentansprüche

1. Intersomatischer Käfig, der geeignet ist, zwischen zwei Knochenabschnitten implantiert zu werden, der Käfig umfassend einen Festkörper (10), der durch zwei gegenüberliegende Anlageflächen (13, 14) begrenzt ist, die durch eine Seitenwand mit Außenoberfläche (15) verbunden sind, wobei diese Anlageflächen mit ebenen Oberflächen vergleichbar sind, die im Wesentlichen parallel zueinander sind, und so angeordnet sind, dass sie geeignet sind, jeweils mit den zwei Knochenabschnitten in Kontakt zu kommen, wobei der Festkörper (10) ferner einen durchgehenden Hohlraum (11) aufweist, der jeweils an seinen zwei Enden in die zwei Anlageflächen (13, 14) so mündet, dass die Mittelachse (12) des Hohlraums (11) im Wesentlichen senkrecht zu diesen zwei Anlageflächen ist; und Mittel zum festen Verbinden des Festkörpers mit mindestens einem der zwei Knochenabschnitte, diese Mittel zum festen Verbinden des Festkörpers (10) mit mindestens einem der zwei Knochenabschnitte umfassend: mindestens eine Trägerplatte (20); Mittel zum Montieren der Trägerplatte in dem Hohlraum (11) in Zusammenwirkung mit dem Festkörper; mindestens eine Verankerungsklinge (30); und Mittel zum Montieren der Verankerungsklinge in Bewegungszusammenwirkung auf der Trägerplatte, die durch Drehbewegungsmittel (40) der Verankerungsklinge in Bezug auf die Trägerplatte so gebildet sind, dass in einer ersten Position die Verankerungsklinge (30) vollständig in dem Hohlraum (11) enthalten ist, und dass in einer zweiten Position nach ihrer Bewegung in Bezug auf die Trägerplatte (20) mindestens ein Teil (31, 32) der Verankerungsklinge (30) aus dem Hohlraum (11) herausragt,
wobei die Mittel zum Montieren der Trägerplatte (20)
in dem Hohlraum (11) in Zusammenwirkung mit dem Festkörper (10) durch Mittel zum Befestigen der Trägerplatte (20) auf dem Festkörper gebildet sind, wobei die Trägerplatte (20) ferner so angeordnet ist, dass sie geeignet ist, verschiedene Winkelausrichtungen in dem Hohlraum in Bezug auf den Festkörper (10) einzunehmen, gemäß mindestens einer der zwei folgenden Drehungen: einer Drehung um die Mittelachse (12), einer Drehung um eine Gerade, die im Wesentlichen senkrecht zu dieser Mittelachse und senkrecht zu der Drehachse der Verankerungsklinge in Bezug auf die Trägerplatte ist.

2. Käfig nach Anspruch 1, **dadurch gekennzeichnet, dass** die Drehbewegungsmittel (40) der Verankerungsklinge (30) in Bezug auf die Trägerplatte (20) eine Welle (41), die mit der Trägerplatte gekoppelt ist, und Mittel zum Montieren der Verankerungsklinge in Drehzusammenwirkung mit der Welle aufweisen.

3. Käfig nach Anspruch 2, **dadurch gekennzeichnet, dass** die Welle (41) in der Trägerplatte (20) drehbar montiert ist und dass die Mittel zum Montieren der Verankerungsklinge (30) in Zusammenwirkung mit der Welle (41) so angeordnet sind, dass die Klinge mit der Welle (41) fest verbunden ist.

4. Käfig nach Anspruch 3, **dadurch gekennzeichnet, dass** die Verankerungsklinge (30) auf der Trägerplatte (20) eng drehbar montiert ist.

5. Käfig nach einem der vorstehenden Ansprüche,
**dadurch gekennzeichnet, dass** die Verankerungsklinge (30) so konfiguriert ist, dass in ihrer zweiten Position mindestens zwei Teile (31, 32) der Klinge jeweils aus den zwei Enden des Hohlraums (11) herausragen.

6. Käfig nach einem der vorstehenden Ansprüche,
**dadurch gekennzeichnet, dass** die Wand des Festkörpers eine Öffnung (50) aufweist, die in den Hohlraum (11) mündet, wobei die Öffnung einen Querschnitt besitzt, der geeignet ist, ein Hilfswerkzeug (An) zum Steuern des Übergangs der Verankerungsklinge von einer ihrer zwei Positionen in die andere übergehen zu lassen, wobei der Querschnitt der Öffnung (50) einen Wert besitzt, der so bestimmt ist, dass das Hilfswerkzeug verschiedene Ausrichtungen (51) in Bezug auf die Mittelachse (12) des Hohlraums (11) einnehmen kann.

7. Käfig nach Anspruch 6, **dadurch gekennzeichnet, dass** er Mittel zum Befestigen der Trägerplatte aufweist, wenn diese eine bestimmte Ausrichtung eingenommen hat.

8. Käfig nach Anspruch 7, **dadurch gekennzeichnet, dass** die Mittel zum Befestigen der Trägerplatte (20), wenn diese eine bestimmte Ausrichtung eingenommen hat, durch eine Sperrzahnstange gebildet sind.

9. Käfig nach einem der vorstehenden Ansprüche,
**dadurch gekennzeichnet, dass** er mindestens zwei Paare "Trägerplatte/Verankerungsklinge (20/30)" aufweist, die in dem Hohlraum (11) in zwei nicht zusammenfallenden Positionen gelegen sind.

## Claims

1. An intersomatic cage suitable for implantation between two bone segments, said cage comprising a solid body (10) defined by two opposing bearing faces (13, 14) connected by a lateral wall with an outer surface (15), these bearing faces being equivalent to plane surfaces substantially parallel to one another and being arranged to come into contact with the two bone segments, respectively, said solid body (10) further comprising a through cavity (11) opening at its two ends onto the two bearing faces (13, 14), respectively, such that the central axis (12) of the cavity (11) is substantially perpendicular to these two bearing faces; and means for securing said solid body to at least one of the two bone segments, these means for securing said solid body (10) to at least one of the two bone segments comprising: at least one support plate (20); means for mounting said support plate in said cavity (11) in engagement with said solid body; at least one anchoring blade (30); and means for mounting said anchoring blade in movable engagement on said support plate, consisting of means (40) for rotational movement of said anchoring blade relative to said support plate, such that, in a first position, said anchoring blade (30) is entirely contained within said cavity (11) and, in a second position following its movement relative to said support plate (20), at least a portion (31, 32) of said anchoring blade (30) protrudes from said cavity (11),
the means for mounting said support plate (20)
in said cavity (11) in engagement with said solid body (10), consisting of means for fastening said support plate (20) to said solid body, said support plate (20) being further arranged so as to be capable of assuming different angular orientations within said cavity relative to the solid body (10), according to at least one of the following two rotations: rotation about the central axis (12), rotation about a line substantially perpendicular to this central axis and perpendicular to the axis of rotation of the anchoring blade relative to the support plate.

2. The cage according to claim 1, **characterized in that** the means (40) for rotational movement of the anchoring blade (30) relative to the support plate (20) comprise a shaft (41) coupled to said support plate and means for mounting said anchoring blade in rotational engagement with said shaft.

3. The cage according to claim 2, **characterized in that** said shaft (41) is rotatably mounted in the support plate (20) **and in that** the means for mounting said anchoring blade (30) in engagement with said shaft (41) are arranged such that said blade is secured to said shaft (41).

4. The cage according to claim 3, **characterized in that** said anchoring blade (30) is rotatably mounted on said support plate (20) in a clamped manner.

5. The cage according to any of the preceding claims,
**characterized in that** said anchoring blade (30) is configured such that, in its second position, at least two portions (31, 32) of said blade protrude from both ends of said cavity (11), respectively.

6. The cage according to any of the preceding claims, **characterized in that** the wall of said solid body includes an opening (50) leading into said cavity (11), said opening having a cross-section capable of allowing an auxiliary tool (An) to pass through in order to control the movement of said anchoring blade from one of its two positions to the other, the cross-section of said opening (50) having a specific value such that the auxiliary tool can assume different orientations (51) relative to said central axis (12) of said cavity (11).

7. The cage according to claim 6, **characterized in that** it comprises means for fastening said support plate when it has assumed a specific orientation.

8. The cage according to claim 7, **characterized in that** the means for fastening said support plate (20) when it has assumed a specific orientation consist of a one-way rack.

9. The cage according to any of the preceding claims,
**characterized in that** it comprises at least two "support plate/anchoring blade (20/30)" pairs located in said cavity (11) in two distinct positions.
